(19) Europäisches Patentamt / European Patent Office / Office européen des brevets

(11) **EP 3 363 913 B1**

(12) **FASCICULE DE BREVET EUROPEEN**

(45) Date de publication et mention de la délivrance du brevet:
**07.06.2023 Bulletin 2023/23**

(21) Numéro de dépôt: **18156065.7**

(22) Date de dépôt: **09.02.2018**

(51) Classification Internationale des Brevets (IPC):
**C12Q 1/6851** (2018.01)    **C12Q 1/6825** (2018.01)

(52) Classification Coopérative des Brevets (CPC):
(C-Sets disponibles)
**C12Q 1/6825; C12Q 1/6851; G01N 21/272;
G01N 21/4788; G01N 21/75; G01N 21/82;**
G01N 2021/755; G03H 2001/0447    (Cont.)

(54) **PROCÉDÉ OPTIQUE DE SUIVI DE L'AMPLIFICATION IN-VITRO D'UNE SÉQUENCE DE NUCLÉOTIDES**

OPTISCHES VERFAHREN ZUR NACHVERFOLGUNG DER IN-VITRO-AMPLIFIKATION EINER NUKLEOTIDSEQUENZ

OPTICAL METHOD FOR MONITORING THE IN VITRO AMPLIFICATION OF A NUCLEOTIDE SEQUENCE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.02.2017 FR 1751291**

(43) Date de publication de la demande:
**22.08.2018 Bulletin 2018/34**

(73) Titulaire: **COMMISSARIAT À L'ÉNERGIE ATOMIQUE ET AUX ÉNERGIES ALTERNATIVES**
**75015 Paris (FR)**

(72) Inventeurs:
• **ALLIER, Cédric**
**38000 GRENOBLE (FR)**
• **BAQUE, Melissa**
**38000 GRENOBLE (FR)**
• **BORDY, Thomas**
**38000 GRENOBLE (FR)**
• **BOURDAT, Anne-Gaëlle**
**38260 NANTOIN (FR)**
• **VELLOU, Daniel**
**38170 SEYSSINET (FR)**

(74) Mandataire: **INNOV-GROUP**
**310, avenue Berthelot**
**69372 Lyon Cedex 08 (FR)**

(56) Documents cités:
**EP-A1- 3 115 770**    **WO-A1-2016/151248**
**WO-A1-2016/189257**    **US-A1- 2016 291 015**

• **Tiantian Wang ET AL: "Real-time DNA Amplification and Detection System Based on a CMOS Image Sensor", Analytical Sciences, 1 janvier 2016 (2016-01-01), pages 653-658, XP055417270, DOI: 10.2116/analsci.32.653 Extrait de l'Internet: URL:https://www.jstage.jst.go.jp/article/a nalsci/32/6/32_653/_pdf [extrait le 2017-10-19]**
• **NARONG ARUNRUT ET AL: "Demonstration of a Very Inexpensive, Turbidimetric, Real-Time, RT-LAMP Detection Platform Using Shrimp Laem-Singh Virus (LSNV) as a Model", PLOS ONE, vol. 9, no. 9, 25 septembre 2014 (2014-09-25), page e108047, XP055417284, DOI: 10.1371/journal.pone.0108047**
• **MORI Y ET AL: "Real-time turbidimetry of LAMP reaction for quantifying template DNA", JOURNAL OF BIOCHEMICAL AND BIOPHYSICAL METHODS, AMSTERDAM, NL, vol. 59, no. 2, 31 mai 2004 (2004-05-31), pages 145-157, XP002339944, ISSN: 0165-022X, DOI: 10.1016/J.JBBM.2003.12.005**

- **MORI YASUYOSHI ET AL: "Detection of loop-mediated isothermal amplification reaction by turbidity derived from magnesium pyrophosphate formation", BIOCHEMICAL AND BIOPHYSICAL RESEARCH COMMUNICATIONS, ELSEVIER, AMSTERDAM, NL, vol. 289, no. 1, 23 novembre 2001 (2001-11-23), pages 150-154, XP002255232, ISSN: 0006-291X, DOI: 10.1006/BBRC.2001.5921**
- **Jens Fischbach ET AL: "Shining a light on LAMP assays--a comparison of LAMP visualization methods including the novel use of berberine", BioTechniques, 1 avril 2015 (2015-04-01), pages 189-194, XP055273889, England DOI: 10.2144/000114275 Extrait de l'Internet: URL:http://www.biotechniques.com/multimedi a/archive/00249/BTN_A_000114275_O_249515a. pdf [extrait le 2016-05-20]**
- **Anonymous: "Imaging Electronics 101: Understanding Camera Sensors for Machine Vision Applications", , 20 February 2016 (2016-02-20), pages 1-7, XP055609583, Retrieved from the Internet: URL:https://web.archive.org/web/2016022017 0831/http://www.edmundoptics.com/resources /application-notes/imaging/understanding-c amera-sensors-for-machine-vision-applicati ons/ [retrieved on 2019-07-30]**

- **Motoki Goto ET AL: "Colorimetric detection of loop-mediated isothermal amplification reaction by using hydroxy naphthol blue", BioTechniques, vol. 46, no. 3, 1 March 2009 (2009-03-01), pages 167-172, XP055032609, ISSN: 0736-6205, DOI: 10.2144/000113072**
- **Laura Zanoli ET AL: "Isothermal Amplification Methods for the Detection of Nucleic Acids in Microfluidic Devices", Biosensors, vol. 3, no. 1, 27 December 2012 (2012-12-27), pages 18-43, XP055412468, DOI: 10.3390/bios3010018**

Remarques:

Le document complet y compris le(s) table(s) de référence et le(s) listage(s) de séquence sont téléchargeables sur le site internet de l'OEB

(52) Classification Coopérative des Brevets (CPC): (Cont.)

C-Sets
**C12Q 1/6825, C12Q 2525/301, C12Q 2563/137, C12Q 2565/301;
C12Q 1/6851, C12Q 2525/301, C12Q 2563/137, C12Q 2565/301, C12Q 2565/607**

**Description**

**DOMAINE TECHNIQUE**

Le domaine technique de l'invention est un procédé de suivi de l'amplification d'une séquence de nucléotides.

**ART ANTERIEUR**

L'amplification sélective d'une séquence de nucléotides, tel un fragment d'ADN ou d'ARN, s'est considérablement développée depuis la mise au point de la méthode de réaction de polymérase en chaîne, usuellement désignée par l'acronyme PCR signifiant "Polymerase Chain Reaction". Une telle méthode permet de disposer de copies d'une séquence de nucléotides initialement présentes dans un échantillon en mettant en oeuvre des procédés à présent bien standardisés. Les applications sont nombreuses, notamment dans le domaine du clonage de fragments d'ADN spécifiques, la détection et l'identification de gènes pour le diagnostic des maladies génétiques, ou pour des applications en médecine légale. Parmi les applications en dehors du domaine de la santé, on peut citer le contrôle de denrées alimentaires, et par exemple la détection de la présence d'ADN génétiquement modifié. Un des inconvénients de la PCR et cependant la nécessité de réaliser des cycles thermiques précis.

Récemment, des méthodes d'amplification alternatives à la PCR sont apparues, pouvant fonctionner dans des conditions isothermes, et ne nécessitant pas la répétition de cycles thermiques. Citons par exemple la méthode désignée par l'acronyme LAMP, signifiant "Loop-mediated isothermal amplification of DNA", apparue depuis les années 2000, et décrite dans la publication Notomi T, "Loop-mediated isothermal amplification of DNA", Nucleic Acids Research, 2000, Vol. 28, N° 12. Cette méthode présente de nombreux avantages, en particulier une possibilité d'une mise en oeuvre à température constante, une spécificité élevée et une efficacité d'amplification élevée. La publication Hill Joshua "Loop-Mediated Isothermal Amplification Assay for Rapid Detection of Common Strains of Escherichia coli", Journal of Clinical Microbiology, Aug. 2008, p. 2800-2804, décrit le recours à une amplification de type LAMP pour la détection de bactéries de type *Escherichia coli.* Cette publication est désignée par la suite "Hill 2008".

Quelle que soit la méthode d'amplification mise en oeuvre, un contrôle de l'avancement de la réaction d'amplification est souvent nécessaire. Pour cela, des méthodes optiques ont été développées, notamment par l'adjonction d'un label fluorescent dans l'échantillon, de façon à effectuer une corrélation entre l'amplification et un niveau de fluorescence détecté. En complément ou en alternative au label fluorescent, un indicateur coloré peut être utilisé, de façon à permettre un suivi optique de l'amplification.

Le recours à un indicateur coloré de type Bleu d'Hydroxynaphthol (HNB) est par exemple préconisé dans la publication Wang T. et al "Real-time DNA Amplification and Détection System based on a CMOS Image sensor". Dans cette publication, la solution dans laquelle a lieu l'amplification est éclairée dans une bande spectrale rouge. Sous l'effet de l'amplification, la couleur de l'échantillon vire progressivement vers le bleu.

Cependant, l'ajout d'agents fluorescents ou de d'indicateurs colorés exogènes peut avoir des conséquences sur le processus d'amplification et/ou peut présenter un risque de toxicité. De plus, cela a un impact sur le coût et la complexité de l'opération. En effet, la détection d'un signal fluorescent nécessite des capteurs sensibles, disposant d'un filtre optique centré sur la longueur d'onde de fluorescence. Les réactifs colorés ou fluorescents génèrent également un surcoût non négligeable, et leur utilisation accroît la complexité de la méthode.

La publication Mori Y. "Real-time turbidimetry of LAMP reaction for quantifying template DNA", J. Biochem. Biophys. Methods 59 (2004) 145-157, décrit un procédé optique de suivi de l'amplification in-vitro d'une séquence d'ADN par une mesure d'une turbidité de l'échantillon. Dans cette publication, une amplification d'un acide nucléique est réalisée par LAMP. L'échantillon est disposé entre une source de lumière et une photodiode. A partir de l'intensité du signal mesuré par la photodiode, on détermine une grandeur représentative d'une turbidité du milieu, dépendant de l'amplification. L'échantillon est placé dans des tubes de 2.5 mm de diamètre, la source de lumière et la photodiode étant diamétralement opposées. La publication Mori Y "Détection of Loop-Mediated Isothermal Amplification Reaction by Turbidity Derived from Magnesium Pyrophosphate Formation", Biochemical and Biophysical Research Communications 289, 150-154 (2001), attribue l'évolution de la turbidité à la formation de pyrophosphate de magnésium durant l'amplification LAMP. Une telle méthode est à présent mise en oeuvre de façon industrielle, par exemple dans le dispositif Loopamp (marque déposée) commercialisé par la société Eiken.

Cependant, cette méthode n'est pas appropriée à des milieux réactionnels trop opaques, par exemple des milieux comportant du sang. En effet, l'absorption est trop importante et le signal mesuré est davantage dominé par l'atténuation optique que par la diffusion résultant de l'évolution de la turbidité du milieu. De plus, la sensibilité de cette méthode est limitée, et nécessite la formation d'une quantité relativement importante de pyrophosphate de magnésium, de façon à obtenir une atténuation significative de la lumière émise par la source. Les inventeurs ont développé un procédé optique de suivi d'une réaction d'amplification d'une séquence de nucléotides sans marquage, présentant une sensibilité accrue par rapport à l'art antérieur. De plus, le procédé est adapté à des milieux réactionnels colorés. Son application est simple et peu coûteuse. Aussi, le procédé peut être implémenté sur des dispositifs compacts et portables.

## EXPOSE DE L'INVENTION

Un premier objet de l'invention est un procédé de suivi de l'amplification in-vitro d'une séquence de nucléotides contenue dans un échantillon, l'amplification visant à répliquer ladite séquence nucléotides par une enzyme ADN polymérase, l'amplification étant de type LAMP (Loop-Mediated Isothermal Amplification), procédé comportant les étapes suivantes :

a) mélange de l'échantillon avec des réactifs dits d'amplification, aptes à l'obtention de réplications multiples d'une séquence de nucléotides prédéterminée, dite séquence cible, le mélange étant réalisé à un instant initial ;
b) illumination de l'échantillon à l'aide d'une source de lumière, de préférence spatialement cohérente, la source de lumière émettant une onde lumineuse incidente se propageant vers l'échantillon, dans une bande spectrale d'illumination;
c) acquisition, à l'aide d'un capteur d'image, d'une image bidimensionnelle de l'échantillon, dans la bande spectrale d'illumination, l'échantillon étant disposé entre la source de lumière et le capteur d'image de telle sorte que le capteur d'image est exposé à une onde lumineuse dite d'exposition ayant traversé l'échantillon, l'acquisition étant réalisée à un instant d'acquisition postérieur à l'instant initial.
d) à partir de l'image acquise, formation d'une image d'intérêt, représentative de la formation d'un précipité dans l'échantillon sous l'effet de l'amplification ;
e) détermination d'un indicateur, dit indicateur d'amplification, en fonction d'une distribution de l'intensité des pixels dans tout ou partie de l'image d'intérêt, l'indicateur d'amplification étant représentatif d'une quantité de séquences cibles répliquée;

le procédé étant caractérisé en ce que lors de l'étape c), l'image bidimensionnelle de l'échantillon est acquise dans la bande spectrale d'illumination et que lors de l'étape e), l'indicateur d'amplification est déterminé en fonction d'une dispersion de l'intensité des pixels dans l'image d'intérêt.

L'indicateur d'amplification représente une dispersion de l'intensité de pixels dans tout ou partie de l'image d'intérêt.

Dans l'étape e), la détermination de l'indicateur d'amplification est obtenue à l'aide d'un indicateur statistique représentant une dispersion d'une distribution de l'intensité des pixels composant l'image d'intérêt. La détermination de l'indicateur d'amplification peut être obtenue par le calcul d'un moment d'ordre strictement supérieur à 1 de ladite distribution. Le moment peut être un moment centré ou un moment centré réduit.

L'indicateur d'amplification peut permettre de déterminer un instant dit instant seuil, à partir duquel l'indicateur d'amplification franchit un seuil prédéterminé.

L'onde lumineuse d'exposition peut notamment être formée d'une partie de l'onde lumineuse incidente, non absorbée par l'échantillon, et d'une onde résultant de la diffraction, par l'échantillon, de l'onde lumineuse incidente. Plus la quantité de précipité dans l'échantillon est importante, plus la diffraction de l'onde lumineuse incidente par l'échantillon est significative, et observable sur l'image acquise par le capteur d'image.

Selon un mode de réalisation, l'image d'intérêt correspond à tout ou partie de l'image acquise. Selon un autre mode de réalisation, l'image d'intérêt est une image obtenue en appliquant un opérateur de reconstruction holographique à tout ou partie de l'image acquise. Dans ce cas, l'étape d) du procédé comporte les sous-étapes suivantes :

di) à partir de tout ou partie de l'image acquise lors de l'étape c), application d'un opérateur de propagation de façon à calculer une amplitude complexe de l'onde lumineuse d'exposition selon une surface de reconstruction s'étendant face au capteur d'image;
dii) à partir de l'amplitude complexe, formation d'une image, dite image reconstruite, représentative du module et/ou de la phase de ladite amplitude complexe dans la surface de reconstruction, l'image reconstruite formant alors l'image d'intérêt.

La surface de reconstruction peut être un plan parallèle à un plan, dit plan de détection, selon lequel s'étend le capteur d'image, et passant de préférence par l'échantillon.

L'indicateur calculé lors de l'étape e) peut être obtenu par l'application d'un indicateur statistique à l'image reconstruite lors de la sous-étape dii). Il peut notamment s'agir d'un moment d'ordre supérieur à 1 de la distribution de l'intensité de pixels de l'image reconstruite, cette dernière formant l'image d'intérêt. De préférence, l'indicateur statistique est obtenu à partir de la variance ou de l'écart type de la distribution.

Les étapes b) à c) sont de préférence mises en oeuvre lors des réplications de la séquence cible. Elles sont généralement mises en oeuvre de façon itérative, pour obtenir une évolution temporelle de l'indicateur d'amplification, ce dernier dépendant de la quantité de séquences cibles répliquées. Ainsi, les étapes a) à e) peuvent être effectuées à différents instants d'acquisition, le procédé comportant alors une étape f) de suivi temporel de la valeur de l'indicateur d'amplification. En effet, l'amplification s'accompagne de la formation d'ions pyrophosphate dans l'échantillon sous l'effet des réplications de la séquence cible. La formation d'un précipité insoluble à partir des ions phosphates influe sur les propriétés de diffusion et de diffraction de la lumière par l'échantillon. La présence de tels précipités peut être mise en évidence sur l'image formée lors de l'étape c). Le précipité formé peut être du pyrophosphate de magnésium.

Le procédé peut comporter une étape g) de détection d'un instant, dit instant seuil, auquel l'indicateur d'amplification franchit une valeur seuil, de façon à obtenir une durée entre l'instant seuil et l'instant initial.

L'échantillon est de préférence contenu dans une chambre fluidique dont l'épaisseur est inférieure à 1 mm, la surface de l'échantillon observée par le capteur d'image étant supérieure à 10 mm$^2$.

De préférence, lors de l'étape a) les réactifs mélangés à l'échantillon en vue d'obtenir une réplication de la séquence cible sont adaptés à la formation d'un précipité à partir des ions pyrophosphates libérés lors des réplications des séquences cibles. Après le mélange, l'échantillon peut en particulier être exempt de pyrophosphatase, ou dans une quantité suffisamment faible pour permettre une formation suffisamment importante d'un précipité à partir des ions pyrophosphates. Par formation suffisamment importante, on entend une concentration d'au moins 0.5 mM, voire d'au moins 1 mM. Après le mélange, l'échantillon comporte des ions permettant la formation d'un précipité insoluble à partir des ions pyrophosphates résultant du processus d'amplification. Il peut par exemple s'agir d'ions manganèse ou d'ions magnésium.

Par séquence de nucléotides, on entend par exemple un segment d'un acide nucléique. L'amplification consiste notamment en l'utilisation d'amorces spécifiques de la séquence cible à amplifier, les amorces étant aptes à s'hybrider de part et d'autre de la séquence à amplifier, et au recours à une enzyme polymérase. L'enzyme polymérase permet la synthèse d'un brin de polynucléotides.

De préférence, aucune optique de grossissement ni de formation d'image n'est disposée entre l'échantillon et le support.

Un deuxième objet de l'invention est un dispositif pour le suivi de l'amplification in-vitro d'une séquence de nucléotides dans un échantillon, le dispositif comportant :

- une source de lumière ponctuelle apte à émettre une onde lumineuse incidente se propageant vers l'échantillon;
- un support, configuré pour maintenir l'échantillon entre la source de lumière et un capteur d'image ;
- un processeur, configuré pour recevoir une image de l'échantillon acquise par le capteur d'image et à mettre en oeuvre les étapes c) à e) du premier objet de l'invention.

Selon un mode de réalisation, aucune optique de formation d'image ou de grossissement n'est disposée entre le capteur d'image et le support.

Un troisième objet décrit mais ne faisant pas partie de l'invention concerne un procédé de suivi de l'amplification in-vitro d'une séquence de nucléotides, contenue dans un échantillon, l'amplification visant à répliquer ladite séquence de nucléotides par une enzyme ADN polymérase, le procédé comportant les étapes suivantes :

i) mélange de l'échantillon avec des réactifs dits d'amplification, aptes à l'obtention de réplications multiples d'une séquence de nucléotides prédéterminée, dite séquence cible, le mélange étant réalisé à un instant initial ;
ii) illumination de l'échantillon à l'aide d'une source de lumière, la source de lumière émettant une onde lumineuse incidente se propageant vers l'échantillon ;
iii) acquisition, à l'aide d'un photodétecteur, d'une onde lumineuse dite d'exposition ayant traversé tout ou partie de l'échantillon, l'acquisition étant réalisée à un instant d'acquisition postérieur à l'instant initial ;
iv) détermination d'un indicateur d'amplification de la séquence cible à partir d'un indicateur, dit indicateur d'amplification, déterminé en fonction d'une intensité de l'onde lumineuse d'exposition détectée lors de l'étape iii) ;

le procédé comportant l'introduction, dans l'échantillon, d'un réactif propice à la formation d'ions pyrophosphates dans ce dernier.

Le réactif peut être un sel de pyrophosphate, par exemple du pyrophosphate de potassium. De préférence, le réactif propice à la formation d'ions pyrophosphates dans l'échantillon est introduit préalablement ou simultanément aux réactifs d'amplification.

Lorsque l'échantillon comporte du magnésium ou du manganèse, cela permet une détection précoce de l'amplification de la séquence de nucléotides cibles.

L'indicateur d'amplification peut quantifier une absorption de l'onde lumineuse incidente par l'échantillon. L'onde lumineuse d'exposition peut être une onde émise dans une bande spectrale différente de la bande spectrale de l'onde d'illumination, par exemple une fluorescence. Selon un mode de réalisation, l'onde lumineuse d'exposition est formée d'une interférence entre l'onde lumineuse incidente non absorbée par l'échantillon et une onde résultant de la diffraction de l'onde lumineuse incidente par l'échantillon.

Selon ce mode de réalisation, le photodétecteur peut être une photodiode ou un capteur d'image. Selon ce mode de réalisation, le procédé peut présenter les caractéristiques du premier objet de l'invention.

La quantité de réactif propice à la formation d'ions pyrophosphates est telle que la concentration d'ions pyrophosphates dans l'échantillon, avant la réplication des séquences cibles, est de préférence comprise entre 0.01 mM et 0.5 mM, et de préférence entre 0.05 mM et 0.5 mM.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

La figure 1 est un exemple de dispositif permettant la mise en oeuvre de l'invention.

La figure 2A représente les principales étapes d'un mode de réalisation. La figure 2B montre les principales étapes d'un autre mode de réalisation.

La figure 3A représente une image d'un échantillon comportant entre 30 et 35 copies d'une séquence cible, l'image étant acquise 40 minutes après le mélange entre l'échantillon et un milieu réactionnel comportant des réactifs d'amplification.

Les figures 3B et 3C représentent des images d'un échantillon comportant entre 300 et 350 copies d'une séquence cible, les images étant respectivement acquises respectivement 38 minutes et 40 minutes après le mélange entre les séquences cibles et un milieu réactionnel comportant des réactifs d'amplification.

La figure 3D illustre une évolution temporelle d'un indicateur d'amplification obtenu à partir d'images de différents échantillons.

La figure 3E montre une relation entre la concentration initiale de l'échantillon en séquence cible et une durée écoulée entre le mélange des séquences cibles avec les réactifs d'amplification et l'obtention d'un indicateur d'amplification dépassant un seuil prédéterminé.

La figure 4A représente une évolution d'un indicateur statistique obtenu à partir d'images d'échantillons comportant du chlorure de manganèse ainsi que différentes concentrations de pyrophosphate de potassium ou de phosphate de potassium. Cet indicateur statistique représentatif d'un indicateur d'amplification. La figure 4B représente une évolution de l'indicateur décrit en lien avec la figure 4A, à partir d'images d'échantillons comportant du sulfate de magnésium ainsi que différentes concentrations de pyrophosphate de potassium ou de phosphate de potassium.

La figure 4C représente l'évolution d'un indicateur d'amplification de deux échantillons comportant une même quantité initiale de séquence cible, un des deux échantillons comportant une quantité initiale de pyrophosphate de potassium obtenue par l'adjonction de pyrophosphate de potassium.

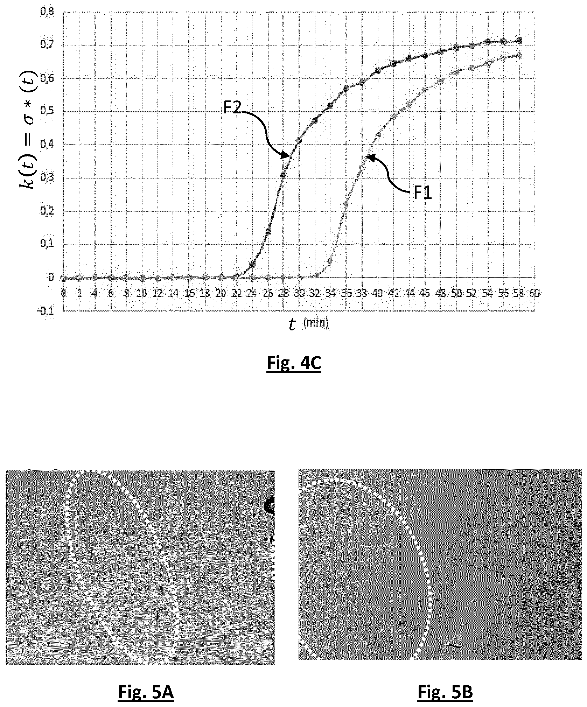

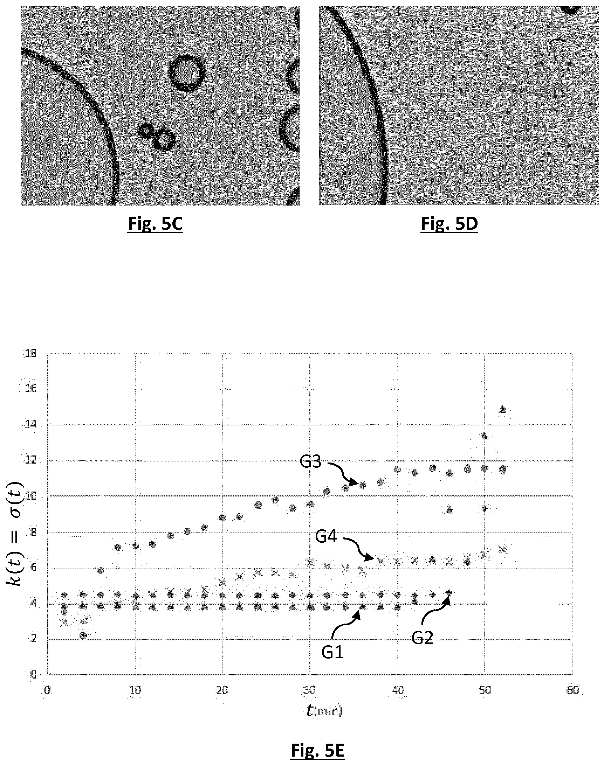

Les figures 5A et 5B représentent des images d'échantillons comportant une même quantité initiale de séquences cibles, obtenues en illuminant ce dernier avec une source de lumière spatialement cohérente, les images étant acquises 38 minutes après le mélange entre les séquences cibles et un milieu réactionnel comportant des réactifs d'amplification. Les figures 5C et 5D représentent des images d'échantillons comportant une même quantité initiale de séquences cibles, les images étant obtenues en illuminant ce dernier avec une source de lumière non spatialement cohérente, les images étant acquises 38 minutes après le mélange entre les séquences cibles et le milieu réactionnel. La figure 5E illustre une évolution temporelle d'un indicateur d'amplification obtenu à partir d'images d'un échantillon respectivement avec ou sans filtre spatial interposé entre la source de lumière et l'échantillon.

## EXPOSE DE MODES DE REALISATION PARTICULIERS

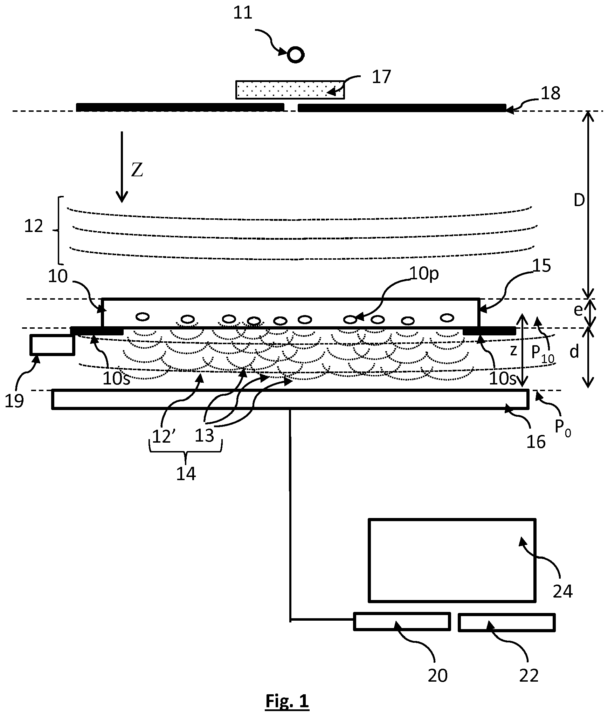

La figure 1 représente un exemple de dispositif selon l'invention. Une source de lumière 11 est apte à émettre une onde lumineuse 12, dite onde lumineuse incidente, se propageant vers un échantillon 10, selon un axe de propagation Z. L'onde lumineuse est émise selon une bande spectrale d'illumination $\Delta\lambda$.

L'échantillon 10 est un échantillon comportant une séquence de nucléotides, dite séquence cible, que l'on souhaite répliquer. Cette séquence de nucléotides peut notamment être un fragment d'ADN. L'échantillon comporte également des réactifs, formant un milieu réactionnel, permettant une réplication de fragments d'ADN, phénomène également désigné par le terme amplification. Le facteur d'amplification, c'est-à-dire le nombre de séquences répliquées à partir d'une séquence cible peut dépasser $10^2$ voire $10^6$ voire davantage. L'échantillon est liquide. Des réactifs d'amplification sont disponibles commercialement, en particulier sous la forme de kits.

L'échantillon 10 est, dans cet exemple, contenu dans une chambre fluidique 15. La chambre fluidique 15 est par exemple une chambre fluidique d'épaisseur e=250 $\mu$m. L'épaisseur e de la chambre fluidique 15, selon l'axe de propagation Z, varie typiquement entre 10 $\mu$m et 1 cm, et est de préférence comprise entre 20 $\mu$m et 500 $\mu$m. L'échantillon 10 s'étend selon un plan $P_{10}$, dit plan de l'échantillon, de préférence perpendiculaire à l'axe de propagation Z. Il est maintenu sur un support 10s à une distance d d'un capteur d'image 16. La surface selon laquelle s'étend l'échantillon, parallèlement au capteur d'image 16, est de préférence supérieure à 1 mm$^2$ et est de préférence supérieure à 5 mm$^2$, voire 10 mm$^2$. Ainsi, de préférence, l'échantillon est peu épais (moins d'1 mm) et s'étend selon une surface de plusieurs mm$^2$. Une telle configuration permet d'accroître la sensibilité du procédé, comme décrit ci-après, en lien avec les essais expérimentaux. Un radiateur 19 permet de maintenir l'échantillon, contenu dans la chambre fluidique, à une température de consigne, par exemple comprise entre 60°C et 65°C.

La distance D entre la source de lumière 11 et l'échantillon 10 est de préférence supérieure à 1 cm. Elle est de préférence comprise entre 2 et 30 cm. Avantageusement, la source de lumière, vue par l'échantillon, est considérée comme ponctuelle. Cela signifie que son diamètre (ou sa diagonale) est préférentiellement inférieur au dixième, mieux au centième de la distance entre l'échantillon et la source de lumière. La source de lumière 11 peut être une diode électroluminescente ou une diode laser. Lorsque la source de lumière est une diode électroluminescente, elle est de préférence associée à diaphragme 18, ou filtre spatial. L'ouverture du diaphragme est typiquement comprise entre 5 μm et 1 mm, de préférence entre 50 μm et 500 μm. Dans cet exemple, le diaphragme est fourni par Thorlabs sous la référence P150S et son diamètre est de 150 μm. Le diaphragme peut être remplacé par une fibre optique, faisant office de filtre spatial, dont une première extrémité est placée face à la source de lumière 11 et dont une deuxième extrémité est placée en regard de l'échantillon 10.

Le dispositif peut comporter un diffuseur 17, disposé entre la source de lumière 11 et le diaphragme 18. L'usage d'un tel diffuseur permet de s'affranchir de contraintes de centrage de la source de lumière 11 par rapport à l'ouverture du diaphragme 18. La fonction d'un tel diffuseur est de répartir le faisceau lumineux, produit par la source de lumière selon un cône d'angle a. De préférence, l'angle de diffusion a varie entre 10° et 80°. La présence d'un tel diffuseur permet de rendre le dispositif plus tolérant à l'égard d'un décentrage de la source de lumière par rapport au diaphragme. Le diaphragme 18 n'est pas nécessaire, en particulier lorsque la source de lumière est suffisamment ponctuelle, notamment lorsqu'il s'agit d'une source laser.

De préférence, la bande spectrale d'illumination $\Delta\lambda$ de l'onde lumineuse incidente 12 a une largeur inférieure à 100 nm. Par largeur de bande spectrale, on entend une largeur à mi-hauteur de ladite bande spectrale.

L'échantillon 10 est disposé entre la source de lumière 11 et le capteur d'image 16 précédemment évoqué. Ce dernier s'étend de préférence parallèlement, ou sensiblement parallèlement au plan $P_{10}$ selon lequel s'étend l'échantillon. Le terme sensiblement parallèlement signifie que les deux éléments peuvent ne pas être rigoureusement parallèles, une tolérance angulaire de quelques degrés, inférieure à 20° ou 10° étant admise.

Le capteur d'image 16 est apte à former une image $I_0$ de l'échantillon selon un plan de détection $P_0$. Dans l'exemple représenté, il s'agit d'un capteur d'image comportant une matrice de pixels, de type CCD ou un CMOS. Le plan de détection $P_0$ s'étend de préférence perpendiculairement à l'axe de propagation Z de l'onde lumineuse incidente 12. La distance $d$ entre l'échantillon 10 et la matrice de pixels du capteur d'image 16 est préférentiellement comprise entre 50 μm et 2 cm, de préférence comprise entre 100 μm et 2 mm. La surface de détection du capteur d'image est de préférence supérieure à 10 mm². L'image est formée dans tout ou partie de la bande spectrale d'illumination. Autrement dit, elle n'inclut pas des composantes spectrales en dehors de la bande spectrale d'illumination.

On remarque l'absence d'optique de grossissement entre le capteur d'image 16 et l'échantillon 10. Cela n'empêche pas la présence éventuelle de microlentilles de focalisation au niveau de chaque pixel du capteur d'image 16, ces dernières n'ayant pas de fonction de grandissement de l'image acquise par le capteur d'image. L'absence d'optique de grossissement permet de disposer le capteur d'image à proximité de l'échantillon, selon la plage de distance décrite dans le paragraphe précédent. Cela augmente la compacité du dispositif.

Préalablement à l'acquisition d'une image exploitable, l'échantillon est mélangé avec des réactifs d'amplification, formant un milieu réactionnel, apte à engendrer une amplification de la séquence cible. Le milieu réactionnel comporte en particulier :

- des amorces, aptes à s'hybrider de part et d'autre de la séquence cible, la longueur typique de ces amorces étant par exemple comprise entre 10 à 50 nucléotides ;
- d'une enzyme polymérase, sachant que de nombreuses enzymes polymérases sont actuellement commercialement disponibles ;
- de solutions de désoxynucléotides ;
- d'un tampon, dont la composition dépend du type et des caractéristiques de l'enzyme utilisée. Le tampon peut notamment comporter des ions magnésium, par exemple sous forme de chlorure de magnésium. Les ions magnésium catalysent l'action de l'enzyme polymérase. Le tampon comporte des ions permettant l'obtention d'un précipité insoluble à partir des ions pyrophosphates formés par l'amplification. Le tampon peut également comporter des ions manganèse.

Selon l'invention, l'amplification de la séquence cible est réalisée par amplification LAMP, telle que décrite en lien avec l'art antérieur. Le fait que l'amplification soit isotherme simplifie le processus et la conception du dispositif mettant en oeuvre l'invention, puisque l'échantillon est porté à une température constante, typiquement entre 60°C et 65°C. Il a été observé, dans l'art antérieur, que la réplication de la séquence cible libère des ions pyrophosphates $P_2O_7^{4-}$. Ces derniers interagissent avec les ions $Mg^{2+}$ pour former un complexe $[Mg]^2[P_2O_7]$ (pyrophosphate de magnésium) apparaissant sous forme de précipité 10p de pyrophosphate de magnésium. La formation de précipités à partir d'autres ions est également possible, par exemple les ions $Mn^{2+}$.

Aussi, plus l'amplification de la séquence cible progresse, plus la concentration de pyrophosphate de magnésium s'élève, ce qui accroît le coefficient de diffusion de l'échantillon ; l'échantillon se trouble et diffuse davantage la lumière. Par ailleurs, plus la concentration de pyrophosphate de magnésium s'élève, plus l'onde lumineuse incidente est diffractée par l'échantillon.

Les inventeurs ont constaté que la formation d'une image de l'échantillon permet une observation précoce de la formation du précipité $10_p$ formé par les ions pyrophosphates, durant le processus d'amplification. Cela permet un suivi visuel de l'amplification selon une surface d'échantillon élevée. Plutôt que de détecter, à l'aide d'une photodiode, une évolution de l'intensité d'un faisceau lumineux traversant une épaisseur d'échantillon de quelques millimètres, comme dans l'art antérieur, les inventeurs ont observé qu'il était préférable de former une image d'un faisceau lumineux traversant une faible épaisseur d'échantillon, l'image étant acquise selon une surface de détection élevée, typiquement supérieure à 5 $mm^2$ et de préférence supérieure 10 $mm^2$. Autrement dit, une configuration faible épaisseur / surface élevée est préférable à la configuration forte épaisseur /faible surface de l'art antérieur.

Sous l'effet de l'onde lumineuse incidente 12, les précipités $10_p$ formés dans l'échantillon peuvent engendrer une onde diffractée 13, susceptible de produire, au niveau du plan de détection $P_0$, des interférences avec une partie de l'onde lumineuse incidente non absorbée par l'échantillon, et transmise par ce dernier. Par ailleurs, l'échantillon 10 peut absorber une partie de l'onde lumineuse incidente 12, en particulier lorsqu'il est coloré. Ainsi, l'onde lumineuse 14, dite onde lumineuse d'exposition, transmise par l'échantillon et à laquelle est exposé le capteur d'image 16, peut comprendre :

- une composante 13 résultant de la diffraction de l'onde lumineuse incidente 12 par l'échantillon, et notamment par les précipités $10_p$ formés dans ce dernier ;
- une composante 12' comportant une partie de l'onde lumineuse incidente 12 par l'échantillon, après une absorption partielle de cette dernière par l'échantillon.

Ces composantes forment des interférences dans le plan de détection. Aussi, l'image acquise par le capteur d'image comporte des figures d'interférences (ou figures de diffraction), ces dernières résultant des précipités 10p formés dans l'échantillon. L'obtention de ces figures de diffraction provient du fait que l'onde lumineuse incidente 12 est produite par une source de lumière spatialement cohérente. Elle atteint l'échantillon sous la forme d'ondes planes. La composante 12' résultant de l'absorption de l'onde lumineuse incidente 12 atteint le capteur d'image 16 sous la forme d'ondes planes, ce qui est propice à l'obtention de figures d'interférence dans le plan de détection.

Un processeur 20, par exemple un microprocesseur, est configuré pour traiter chaque image acquise $I_0$ par le capteur d'image 16. En particulier, le processeur est un microprocesseur relié à une mémoire programmable 22 dans laquelle est stockée une séquence d'instructions pour effectuer les opérations de traitement d'images et de calculs décrites dans cette description. Le processeur peut être couplé à un écran 24 permettant l'affichage d'images acquises par le capteur d'image 16 ou traitées par le processeur 20.

L'utilisation d'une chambre fluidique 15 de faible épaisseur, typiquement inférieure à 1 mm, et de préférence inférieure à 500 $\mu$m, voire 100 $\mu$m, est particulièrement adaptée à des échantillons colorés, par exemple des échantillons comportant du sang. La faible épaisseur de la chambre permet de minimiser l'absorption de la lumière traversant l'échantillon. Par ailleurs, l'acquisition d'une image $I_0$ selon une surface élevée, supérieure à 5 $mm^2$ ou 10 $mm^2$, permet d'identifier une région d'intérêt $ROI$, ou plusieurs régions d'intérêt, propices à la détermination d'un indicateur d'amplification $k$. L'identification de régions d'intérêt dans l'image acquise $I_0$ permet de détecter précocement la formation de précipités $10_p$ dans la chambre fluidique 15, traduisant une amplification de la séquence cible.

Selon un premier mode de réalisation, à partir de l'image $I_0$ acquise par le capteur d'image 16, on forme une image $I$ dite image d'intérêt. L'image d'intérêt $I$ correspond à l'image acquise $I_0$ ou à une région d'intérêt $ROI$ sélectionnée dans l'image acquise $I_0$. Les inventeurs ont observé qu'il était préférable de recourir à des indicateurs exprimant une dispersion de l'intensité des pixels de l'image d'intérêt, par rapport à des indicateurs basés sur une moyenne ou une médiane de l'intensité des pixels de l'image. Une telle dispersion peut être quantifiée par une variance ou un écart-type, ou, de façon plus générale, à partir d'un moment d'ordre supérieur à 1 d'une distribution de l'intensité des pixels de l'image d'intérêt. L'indicateur de dispersion peut être établi à partir d'un moment de la distribution, en particulier à partir d'un moment $m_r$ d'ordre $r$ strictement supérieur à 1 de la distribution. L'indicateur de dispersion peut par exemple être obtenu à partir du coefficient s'asymétrie (moment centré réduit d'ordre 3, connu par le terme anglosaxon skewness) ou à partir du kurtosis (moment centré réduit d'ordre 4).

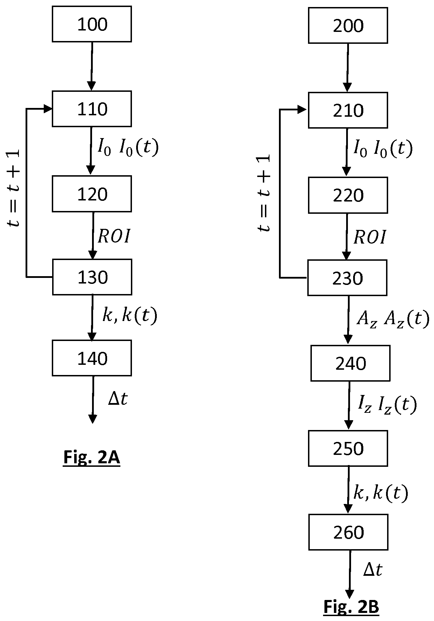

La figure 2A résume les principales étapes du procédé selon ce premier mode de réalisation.

Etape 100 : préparation de l'échantillon : au cours de cette étape, l'échantillon 10, comportant une quantité initiale de séquences cibles, est mélangé à un milieu réactionnel permettant l'amplification de la séquence cible de nucléotides, ainsi que la formation d'un précipité insoluble $10_p$, puis introduit dans la chambre fluidique 15. L'instant $t_0$ correspondant au mélange est un instant dit initial.

Etape 110 : acquisition d'image. L'échantillon 10 est illuminé par la source de lumière 11 et une image $I_0$ est acquise par le capteur d'image 16. L'image est acquise dans la bande spectrale d'illumination, ou dans tout ou partie de la bande spectrale d'illumination.

Etape 120 : sélection d'une zone d'intérêt. Une région d'intérêt $ROI$ est sélectionnée dans l'image acquise. Cette étape est facultative, la région d'intérêt pouvant s'étendre à toute l'image acquise.

Etape 130: détermination d'un indicateur d'amplification k à partir de l'image $I_0$ acquise lors de l'étape 110 ou de la zone d'intérêt *ROI* sélectionnée lors de l'étape 120. L'indicateur d'amplification *k* est de préférence représentatif d'une texturation de l'image ou d'une dispersion de l'intensité des pixels de l'image. Il peut en particulier être obtenu à partir d'un moment de la distribution des pixels et plus particulièrement un moment d'ordre strictement supérieur à 1.

Les étapes 110 à 130 peuvent être réitérées de façon à obtenir, à différents instants *t*, des images acquises $I_0(t)$, des images d'intérêt $I(t)$, à partir de chaque image d'intérêt étant déterminé un indicateur d'amplification $k(t)$. Cela permet d'obtenir une évolution temporelle du facteur d'amplification $k(t)$. On peut alors déterminer un instant dit instant seuil $t_s$, auquel l'indicateur d'amplification franchit une valeur seuil $k_{th}$. La valeur seuil $k_{th}$ peut être une valeur fixe prédéterminée, par exemple au cours d'essais sur des échantillons étalons connus. La durée $\Delta t$ entre l'instant seuil $t_s$ et l'instant initial $t_0$ dépend de la quantité des séquences cibles à l'instant initial. Plus la durée $\Delta t$ est élevée, moins la quantité initiale de séquences cibles est importante. La détermination de l'instant seuil $t_s$ fait l'objet de l'étape 140.

Une série d'essais a été réalisée, en utilisant un échantillon d'une solution d'ADN purifié, extrait à partir d'une culture d'*Escherrichia Coli* disposé dans une chambre fluidique Countess® d'épaisseur 250 $\mu$m, le volume examiné atteignant 8 mm$^3$, soit 8 $\mu$l. La séquence de nucléotides cible est le gène *E coli mal B*.

Le milieu réactionnel comporte :

- un tampon fourni par NEB sous la référence B0537S, comportant 20 mM de tampon Tris-HCl, 50 mM KCl, 10 mM $(NH_4)_2SO_4$, 2 mM MgSO4, 0.1 % Tween (marque déposée) 20, le pH étant de 8.8 à 25°C ;
- 1.2 mM d'un kit de solutions de désoxyribonucléotides triphosphates libres (dNTP), fourni par Sigma sous la référence DNTP100A-1KT ;
- 0.32 U/$\mu$l d'enzyme ADN polymérase *Bst3* (*Bacillus Stearothermophilus*), U significant unité enzymatique ;
- 0.8 M de bétaïne (Sigma B0300) ;
- 4mM MgSO$_4$ (Sigma B1003S), de façon à obtenir des ions Mg$^{2+}$ dans le milieu réactionnel.

Les amorces utilisées sont décrites dans la publication [Hill 2008] citée dans l'art antérieur, et décrites dans le listage annexé à la description. Il s'agit de :

- GCCATCTCCTGATGACGC (extérieur sens, correspondant à la désignation anglosaxone outer forward - concentration 0.2 $\mu$M) ;
- ATTTACCGCAGCCAGACG (extérieur anti-sens, correspondant à la désignation anglosaxone outer backward - concentration 0.2 $\mu$M) ;
- CTGGGGCGAGGTCGTGGTATTCCGACAAA-CACCACGAATT (intérieur sens, correspondant à la désignation anglosaxone inner forward -concentration 1.6 $\mu$M) ;
- CATTTTGCAGCTGTACGCTCGCAGCCCATCA-TGAATGTTGCT (intérieur anti-sens, correspondant à la désignation anglosaxone inner backward - concentration 1.6 $\mu$M) ;
- CTTTGTAACAACCTGTCATCGACA (boucle sens, correspondant à la désignation anglosaxonne loop forward - concentration 0.8 $\mu$M) ;
- ATCAATCTCGATATCCATGAAGGTG (boucle anti-sens, correspondant à la désignation anglosaxonne loop backward - concentration 0.8 $\mu$M).

Une solution de base est élaborée, comportant une quantité d'environ 10$^6$ copies de la séquence cible par microlitre. Une telle quantité est évaluée par une méthode de référence (méthode Qubit - marque déposée).

Les autres éléments du dispositif expérimental sont les suivants :

- source de lumière 11 : diode électroluminescente Créé MC-E Color, comportant trois diodes électroluminescentes émettant respectivement dans les bandes spectrales $\Delta\lambda$ suivantes : 450 nm - 465 nm ; 520 nm - 535 nm ; 620 nm - 630 nm. Dans cet exemple, une seule diode est activée lors de chaque illumination, selon la bande spectrale 450 nm - 465 nm.
- capteur d'image 16 : Capteur CMOS monochrome 3840 x 2748 pixels, chaque pixel mesurant 1.67 $\mu$m de côté, la surface de détection s'étendant sur environ 30 mm$^2$, le champ observé étant de 6.4 mm x 4.6 mm.
- distance D entre la source de lumière 11 et l'échantillon 10 : 5 cm ;
- distance d entre l'échantillon 10 et le capteur d'image 16 : 2mm ;
- épaisseur e de la chambre fluidique 15 : 250 $\mu$m ;
- diamètre de l'ouverture du filtre spatial 18 : 150 $\mu$m ;
- température de l'échantillon : 65 °C.

L'échantillon a été mélangé avec le milieu réactionnel à l'instant initial $t_0$, puis l'ensemble a été introduit dans la chambre fluidique 15.

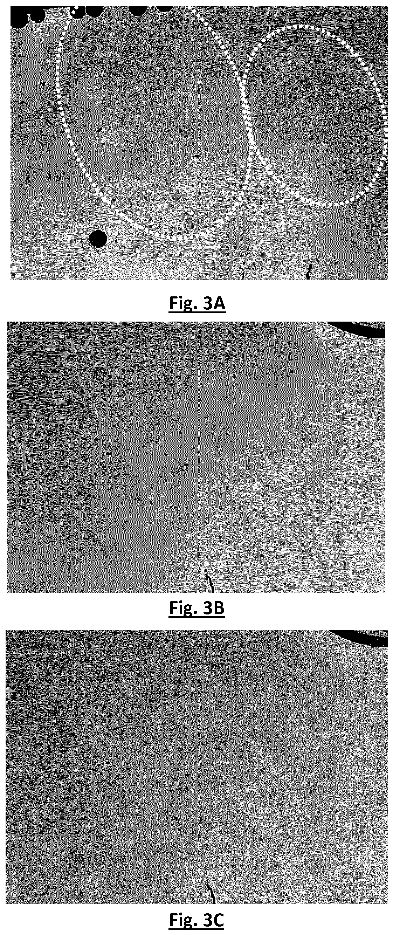

La figure 3A est une image acquise par le capteur d'image 16 quarante minutes après le mélange d'un premier échantillon avec le milieu réactionnel. Le premier échantillon comporte initialement 100 copies de la séquence cible dans un volume de 25 $\mu$l (microlitres). Le volume observé peut être estimé à 8 $\mu$l, ce qui correspond à environ 30 à 35 copies. On observe l'apparition de deux régions d'intérêt *ROI,* entourées par un cercle, dans lesquelles une granularité se forme sur l'image. La texturation de l'image est attribuée à la formation de précipités 10$_p$ de pyrophosphate de magnésium dans l'échantillon. L'acquisition d'une image permet d'identifier des régions d'intérêt dans lesquelles une évolution de la morphologie de l'image est observée. On peut ainsi observer l'amplification de la séquence cible à un stade plus précoce que selon l'art antérieur.

La figure 3B est une image acquise par le capteur d'image 16 trente-huit minutes après le mélange d'un deuxième échantillon avec le milieu réactionnel. Le deuxième échantillon comporte 1000 copies de la séquence cible dans un volume de 25 μl. Le volume observé peut être estimé à 8 μl, ce qui correspond à environ 300 à 350 copies. L'image est relativement uniforme. La formation des précipités de pyrophosphate n'est pas encore observable sur l'image.

La figure 3C est une image acquise par le capteur d'image 40 minutes après le mélange du deuxième échantillon avec le milieu réactionnel. La texturation de l'image est plus nette que sur l'image 3B, acquise seulement 2 minutes auparavant.

Les inventeurs ont observé que l'utilisation de moments, et en particulier la variance ou l'écart type, permet de quantifier l'évolution de la turbidité de l'image résultant de la formation des précipités de pyrophosphate de magnésium sous l'effet du processus d'amplification. A chaque image peut être associé un indicateur, dit indicateur d'amplification, calculé à partir de tels moments. Des essais ont été entrepris avec différents échantillons, présentant une quantité initiale de séquence cible respectivement évaluée à 21 copies (échantillons A1 et A2), 210 copies (échantillons B1 et B2), 2100 copies (échantillons C1 et C2), 21000 copies (échantillons D1 et D2), 210000 copies (échantillons E1 et E2). Des essais ont également été réalisés sur deux échantillons dit de contrôle négatif, comportant le milieu réactionnel sans séquence de nucléotides cible (échantillons N1 et N2). Les images ont été acquises selon une fréquence d'acquisition de 1 image par minute. Sur chaque image acquise, une région d'intérêt est sélectionnée visuellement, et on détermine l'écart type d'une distribution des niveaux de gris des pixels dans la région d'intérêt. Lorsque la quantité initiale de séquences cibles dépasse 20000 copies, la région d'intérêt s'étend à toute l'image acquise.

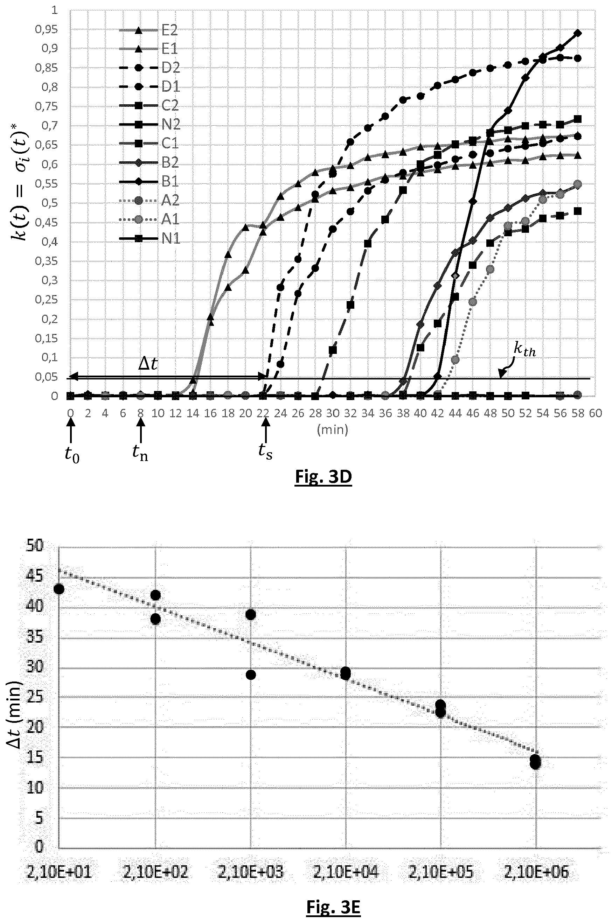

La figure 3D représente l'évolution temporelle d'un l'écart type normalisé sur différentes images acquises, sur chaque échantillon analysé. L'axe des abscisses de cette figure est le temps écoulé depuis l'instant initial $t_0$, exprimé en minutes. L'axe des ordonnées représente une valeur d'un écart type dit normalisé $\sigma_i(t)^*$, obtenu à partir des images acquises par le capteur d'image observant un échantillon $i$, cet écart type étant défini par l'expression suivante :

$$k(t) = \sigma_i(t)^* = \frac{\sigma_i(t) - \overline{\sigma_i(t_0, t_n)}}{\sigma_{Max} - \sigma_{min}}$$

avec:

-   $\sigma_i(t)$ : écart type d'une image acquise à un instant $t$ pour un échantillon $i$ ;
-   $\overline{\sigma_i(t_0, t_n)}$ : écart type moyen d'images acquises entre l'instant initial $t_0$ et un instant dit de proche $t_n$, l'instant

proche étant suffisamment proche de l'instant initial pour que l'image acquise n'évolue pas significativement entre l'instant initial $t_0$ et l'instant proche $t_n$ sous l'effet de l'amplification. Dans cet exemple, l'instant proche $t_n$ est égal à 8 minutes après l'instant initial. Cet écart type moyen permet de prendre en compte les fluctuations de l'image acquise sous l'effet du bruit du capteur d'image 16 ou des fluctuations de la source de lumière 11.

-   $\sigma_{Max}$ est l'écart type maximal mesuré sur l'ensemble des images de l'ensemble des échantillons considérés.
-   $\sigma_{min}$ est l'écart type minimal mesuré sur l'ensemble des images de l'ensemble des échantillons considérés.
-   $i$ désigne l'échantillon.

Pour chaque quantité considérée de séquences cibles, deux échantillons différents ont été examinés. On observe qu'il est possible de détecter une amplification lorsque la quantité initiale de séquences cibles est faible, par exemple 20 copies dans 8 μl. La durée $\Delta t$ au delà de laquelle l'amplification est détectée dépend théoriquement de la quantité initiale de séquences cibles. Sur la figure 3D, cette durée peut être déterminée en identifiant l'instant seuil $t_s$ auquel l'indicateur d'amplification $k(t)$ considéré, en l'occurrence l'écart-type normalisé, dépasse une valeur seuil prédéterminée $k_{th}$. L'instant initial $t_0$ correspond au mélange entre l'échantillon et le milieu réactionnel. Dans cet exemple, la valeur seuil $k_{th}$ est fixée à 0.05. On observe que lorsque la quantité initiale de séquences cibles est faible, typiquement inférieure à 2.1 $10^3$ copies, l'instant $t_s$ de franchissement du seuil ne peut pas être précisément relié à une quantité initiale. Aussi, pour les faibles quantités initiales de séquences cibles, la méthode mise en oeuvre est qualitative : elle permet de mettre en évidence une amplification d'une valeur cible, sans pour autant permettre une quantification précise de la quantité initiale de la séquence cible.

Lorsque la quantité initiale augmente, par exemple pour 2.1 $10^4$, 2.1 $10^5$ ou 2.1 $10^6$ copies, la détection est quantitative, l'instant $t_s$ de franchissement du seuil étant corrélé avec la quantité de séquence cible initialement présente dans l'échantillon. Un exemple de durée de franchissement $\Delta t$ et d'instant de franchissement $t_s$ est représenté sur la figure 3D, en lien avec l'échantillon D1.

Ces essais montrent que l'écart type de chaque image acquise forme un indicateur d'amplification exploitable.

La figure 3E représente l'évolution de la durée $\Delta t$ de franchissement du seuil en fonction de la quantité initiale de séquences cibles dans l'échantillon.

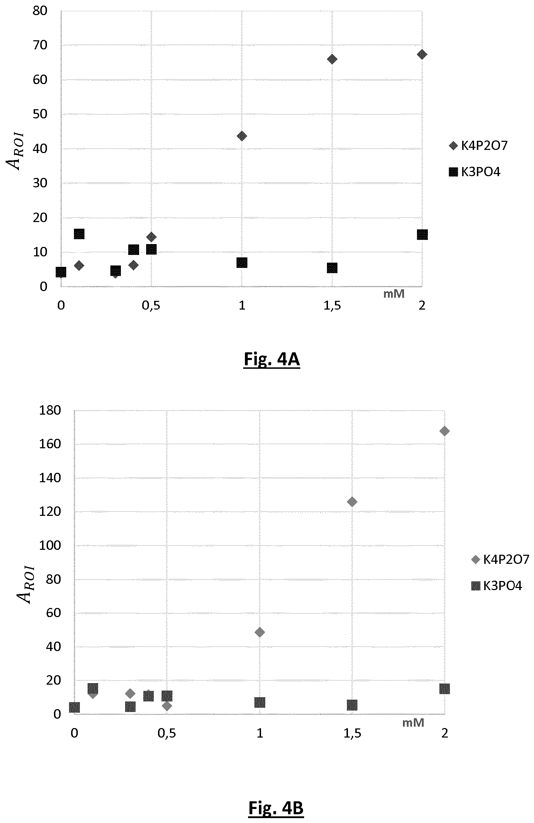

Au cours d'une autre série d'essais, les inventeurs ont vérifié qu'une évolution de la turbidité d'un échantillon pouvait être observée sous l'effet de la formation d'autres composés que le pyrophosphate de magnésium. Aussi, le dispositif précédemment décrit a été mis en oeuvre pour détecter la formation de pyrophosphate de manganèse $MnP_2O_7$. Différents échantillons comportant une solution aqueuse de chlorure de manganèse $Cl_2Mn$, de concentration 4mM, ont été observés par le dispositif. Les échantillons comportent soit du phosphate de potassium ($K_3PO_4$), soit du pyrophosphate de potassium ($K_4P_2O_7$), la concentration en $K_3PO_4$ ou en $K_4P_2O_7$ variant entre 0 et 2 mM. Un précipité impliquant le manganèse ne se forme qu'en présence de pyrophosphate de potassium, le phosphate de potassium étant un monophosphate. Une image a été acquise pour chaque échantillon. Sur chaque image acquise, on a délimité une ou plusieurs régions d'intérêt correspondant à une partie structurée de l'image, de façon analogue à la figure 3A. On a ensuite déterminé l'aire $A_{ROI}$ des régions d'intérêt sélectionnées relativement à la surface totale de l'image. La figure 4A représente les résultats obtenus : l'axe des abscisses correspond à la concentration en $K_3PO_4$ ou en $K_4P_2O_7$, l'axe des ordonnées représentant l'aire relative des régions d'intérêt $A_{ROI}$ préalablement définie. On observe bien, en présence de pyrophosphate de potassium, une formation d'un précipité de pyrophosphate de manganèse ($MnP_2O_7$) lorsque la concentration en $K_4P_2O_7$ dépasse 0.5 mM.

La même expérience a été reconduite, en utilisant des échantillons comportant une concentration 4 mM de sulfate de magnésium ($MgSO_4$). Les échantillons comportent soit du phosphate de potassium ($K_3PO_4$), soit du pyrophosphate de potassium ($K_4P_2O_7$), la concentration en $K_3PO_4$ ou en $K_4P_2O_7$ variant entre 0 et 2 mM. De même que dans l'expérience précédente, un précipité impliquant le magnésium ne se forme qu'en présence de pyrophosphate de potassium. De façon similaire à ce qui a été décrit en lien avec la figure 4A, une image a été acquise pour chaque échantillon. Sur chaque image acquise, on a délimité une ou plusieurs régions d'intérêt correspondant à une partie structurée de l'image. On a ensuite déterminé l'aire $A_{ROI}$ des régions d'intérêt relativement à la surface de l'image. La figure 4B représente les résultats obtenus ; l'axe des abscisses correspond à la concentration en $K_3PO_4$ ou en $K_4P_2O_7$, l'axe des ordonnées représentant l'aire relative des régions d'intérêt $A_{ROI}$. On observe bien, en présence de pyrophosphate de potassium, une formation d'un précipité de pyrophosphate de magnésium lorsque la concentration en $K_4P_2O_7$ dépasse 0.5 mM.

Cela montre que le dispositif permet l'observation de faibles quantités de précipités de pyrophosphate, supérieures ou égales à 0.5 mM ou 1 mM qu'il s'agisse de pyrophosphate de magnésium ou de pyrophosphate de manganèse.

Selon un mode de réalisation, le milieu réactionnel comporte une quantité initiale d'ions pyrophosphates, par exemple selon une concentration de préférence inférieure à 0.5 mM, par exemple 0.4 mM. Ces ions peuvent résulter de l'introduction, dans l'échantillon, d'un sel de pyrophosphate, par exemple de $K_4P_2O_7$. Cela permet de réduire la quantité d'ions pyrophosphates résultant de l'amplification et permettant une détection optique de ladite amplification, par exemple à l'aide de l'indicateur d'amplification préalablement décrit. Le franchissement du seuil $k_{th}$, évoqué en lien avec la figure 3D, est atteint plus rapidement. Cela permet d'abaisser la limite de détection de la méthode, c'est-à-dire de diminuer la quantité initiale de séquences cibles dont l'amplification peut être observable, dans une durée déterminée, sur une image de l'échantillon. Cela permet également d'observer plus rapidement le processus d'amplification. La figure 4C représente une évolution temporelle de l'écart type d'images acquises sur deux échantillons F1 et F2 comportant une quantité initiale de 22 copies par microlitre. L'échantillon F2 a fait l'objet d'un ajout préalable d'une quantité de 0.4 mM de $K_4P_2O_7$. De même que dans les essais présentés sur la figure 3D, des images de chaque échantillon ont été acquises selon une fréquence d'acquisition de 1 image par minute, et on a déterminé l'écart type normalisé des images comme précédemment décrit. La figure 4C montre l'évolution temporelle de l'écart type normalisé, ce dernier faisant office d'indicateur de dispersion. On observe que sur l'échantillon F2, comportant la quantité initiale de pyrophosphate de potassium, l'indicateur de dispersion s'accroît plus rapidement que sur l'échantillon F1. Ainsi, l'ajout préalable de pyrophosphate de potassium permet d'améliorer la rapidité de détection de la méthode ainsi que la limite de détection. Précisons qu'un tel effet ne se limite pas à une méthode d'amplification de type LAMP; mais peut se généraliser aux méthodes d'amplification sous réserve qu'elles s'accompagnent de la formation de précipités. C'est en particulier le cas de la méthode PCR qui ne fait pas partie de la présente invention.

Dans une autre série d'essais, des images ont été acquises, respectivement avec et sans filtre spatial 18 disposé entre la source de lumière 11 et la chambre fluidique 15. Les figures 5A et 5B représentent des images acquises respectivement à l'aide de deux échantillons G1 et G2 tels que précédemment décrit, comportant une quantité initiale de 100 copies de la séquence cible. Ces images ont été obtenues 38 minutes après le mélange entre le milieu réactionnel et les séquences cibles. Le dispositif comporte un filtre spatial prenant la forme d'un diaphragme 18 de type sténopé tel que décrit en lien avec la figure 1. Les figures 5C et 5D représentent des images acquises respectivement à l'aide de deux échantillons G3 et G4, identiques aux échantillons G1 et G2, 38 minutes après avoir été mélangé au milieu réactionnel, sans filtre spatial disposé 18 entre la source de lumière 11 et la chambre fluidique 15. Selon une telle configuration, la source de lumière n'est pas spatialement cohérente. L'image obtenue traduit simplement l'effet de l'atténuation de la lumière par l'échantillon mélangé au milieu réactionnel. Comme on peut l'observer en comparant d'une part les figures 5A et 5B, et d'autre part les figures 5C et 5D, lorsque la source de lumière 11 n'est pas spatialement cohérente, c'est-à-dire dans la configuration liée aux figures 5C et 5D, l'image n'apporte pas d'information exploitable. Au contraire, sur les images 5A et 5B, une structuration de l'image est clairement observable dans les zones d'intérêt entourées, du fait de la formation de figures de diffraction par les précipités $10_p$ résultant du processus d'amplification. Sur les figures 5C et 5D, les zones entourées d'un trait sombre correspondent à des bulles d'air.

Les échantillons G1, G2, G3 et G4 ont chacun fait l'objet d'une série d'acquisition d'image : deux séries d'images ont été acquises avec un filtre spatial 18 (échantillons G1 et G2) entre la chambre fluidique 15 et la source de lumière 11, et deux séries d'image ont été acquises sans filtre spatial (échantillons G3 et G4). Sur chaque image, on a calculé un écart type, faisant office d'indicateur d'amplification. La figure 5E montre l'évolution temporelle de l'indicateur d'amplification $k(t)$ correspondant à l'écart type de chaque image acquise. On observe qu'en mettant en oeuvre le filtre spatial (échantillons G1 et G2), l'instant seuil, auquel l'indicateur d'amplification dépasse une valeur seuil peut être clairement défini : l'évolution temporelle suit une partie plane, jusqu'à t = 45 minutes, puis connaît une croissance brusque. Sans filtre spatial (échantillons G3 et G4), l'évolution de l'indicateur d'amplification est moins nette, et ne présente pas l'inflexion observée en mettant en oeuvre le filtre spatial.

Ces essais montrent que le procédé est essentiellement basé sur la détection spatialisée de figures de diffraction formées par les précipités $10_p$, à la différence des procédés de l'art antérieur reposant sur une atténuation d'un faisceau lumineux incident. Le procédé selon l'invention permet une détection plus précoce des précipités résultant de l'amplification de la séquence cible.

Selon un mode de réalisation, l'indicateur de suivi de l'amplification est établi à partir d'une image d'intérêt résultant de l'application d'un opérateur de propagation numérique à partir de l'image acquise $I_0$. En effet, l'image acquise peut ne pas représenter suffisamment l'échantillon et une représentation plus exploitable peut être obtenue, à partir de l'image acquise, en appliquant un opérateur de propagation de façon à calculer une grandeur représentative de l'onde lumineuse d'exposition 14 à laquelle est exposé le capteur d'image 16. Un tel procédé, désigné par le terme reconstruction holographique, permet notamment de reconstruire une image du module ou de la phase de cette onde lumineuse 14 dans un plan de reconstruction parallèle au plan de détection $P_0$, et notamment dans le plan $P_{10}$ selon lequel s'étend l'échantillon. Pour cela, on effectue un produit de convolution de l'image $I_0$ acquise par le capteur d'image 16 par l'opérateur de propagation $h$. Il est alors possible de calculer une expression complexe $A$ de l'onde lumineuse 14 en tout point de coordonnées $(x,y,z)$ de l'espace, et en particulier dans une surface de reconstruction s'étendant face au capteur d'image. La surface de reconstruction peut notamment être un plan de reconstruction $P_z$ situé à une distance $|z|$ du capteur d'image 16, ce plan de reconstruction étant de préférence un plan $P_{10}$, selon lequel s'étend l'échantillon, avec : $A(x,y,z) = I_0(x,y,z) * h$, *désignant l'opérateur produit de convolution.

Dans la suite de cette description, les coordonnées $(x,y)$ désignent une position radiale dans un plan perpendiculaire à l'axe de propagation Z. La coordonnée z désigne une coordonnée selon l'axe de propagation Z.

L'expression complexe $A$ est une grandeur complexe dont l'argument et le module sont respectivement représentatifs de la phase et de l'intensité de l'onde lumineuse 14 à laquelle est exposé le capteur d'image 16. Le produit de convolution de l'image $I_0$ par l'opérateur de propagation $h$ permet d'obtenir une image complexe $A_z$ représentant une distribution spatiale de l'expression complexe $A$ dans le plan de reconstruction $P_z$, s'étendant à une coordonnée z du plan de détection $P_0$. Dans cet exemple, le plan de détection $P_0$ a pour équation z = 0. L'image complexe $A_z$ correspond à une image complexe de l'échantillon dans le plan de reconstruction $P_z$. Elle représente également une distribution spatiale bidimensionnelle des propriétés optiques de l'onde lumineuse d'exposition 14. L'opérateur de propagation $h$ a pour fonction de décrire la propagation de la lumière entre le capteur d'image 16 et un point de coordonnées $(x,y,z)$, situé à une distance $|z|$ du capteur d'image. Il est alors possible de déterminer le module $M(x,y,z)$ et/ou la phase $\varphi(x,y,z)$ l'onde lumineuse d'exposition 14, à cette distance $|z|$, dite distance de reconstruction, avec :

-
$$M(x,y,z) = abs\,[A(x,y,z)];$$

-
$$\varphi(x,y,z) = arg\,[A(x,y,z)]\,;$$

Les opérateurs *abs* et *arg* désignent respectivement le module et l'argument.

Autrement dit, l'expression complexe $A$ de l'onde lumineuse d'exposition 14 en tout point de coordonnées $(x,y,z)$ de l'espace est telle que : $A(x,y,z) = M(x,y,z)e^{j\varphi(x,y,z)}$. Il est possible de former des images $M_z$ et $\varphi_z$ représentant respectivement une distribution du module ou de la phase de l'expression complexe $A$ dans une surface s'étendant face au plan de détection $P_0$. Une telle surface peut notamment être un plan $P_z$ situé à une distance $|z|$ du plan de détection $P_0$, avec $M_z = \mathrm{mod}(A_z)$ et $\varphi_z = \arg(A_z)$. La surface précédemment évoquée n'est pas forcément plane, mais elle s'étend de préférence parallèlement au plan de détection et est de préférence un plan $P_z$ parallèle au plan de détection. Dans la suite de la description, l'image obtenue à partir du module et/ou de la phase de l'image complexe $A_z$ est désignée par le terme "image reconstruite" et est notée $I_z$.

Les principales étapes de ce mode de réalisation sont présentées sur la figure 2B. Les étapes 200, 210 et 220 sont respectivement identiques aux étapes 100, 110 et 120 préalablement décrites. L'étape 220 de sélection d'une région d'intérêt est facultative, les étapes 230 à 250 s'appliquant aussi bien à toute l'image acquise par le capteur d'image qu'à une région d'intérêt sélectionnée.

Etape 230 : calcul d'une calcul d'une image complexe $A_Z$ dans une surface de reconstruction, idéalement un plan de reconstruction $P_Z$, s'étendant face au capteur d'image. L'image complexe comporte des informations de phase et d'amplitude de l'onde lumineuse d'exposition 14 à laquelle est exposé le capteur d'image 16. Le plan de reconstruction est un plan avantageusement perpendiculaire à l'axe de propagation Z, et/ou parallèle au plan de détection $P_0$. Il s'agit de préférence d'un plan $P_{10}$ selon lequel s'étend l'échantillon. L'étape 230 peut être est effectuée en appliquant l'opérateur de propagation $h$, précédemment décrit, à une image issue de l'image acquise $I_0$, ou à la région d'intérêt $ROI$ sélectionnée. Cependant, l'application de l'opérateur de propagation à l'image acquise peut aboutir à une image complexe $A_Z$ affectée d'un bruit de reconstruction important, fréquemment désigné par le terme twin image. Afin d'obtenir une image complexe exploitable, en limitant le bruit de reconstruction, des algorithmes itératifs peuvent être mis en oeuvre. Un exemple d'algorithme est décrit dans la demande de brevet FR1652500 déposée le 23 mars 2016. L'image complexe $A_Z$ représente les différentes valeurs de l'amplitude complexe $A(x,y,z)$ dans le plan de reconstruction $P_Z$.

Etape 240 : formation d'une image d'intérêt, représentative de l'échantillon. A partir de l'image complexe $A_Z$, on peut obtenir une image reconstruite $I_Z$ à partir du module $M_Z$ et/ou de la phase $\varphi_Z$ de l'onde lumineuse d'exposition 14, dans le plan de reconstruction $P_Z$. La distance de reconstruction par rapport au capteur d'image est déterminée soit a priori, connaissant la position de l'échantillon 10 par rapport au capteur d'image 16, soit sur la base d'un algorithme de focalisation numérique, selon lequel la distance de reconstruction optimale est celle pour laquelle l'image reconstruite $I_Z$ est la plus nette. Les algorithmes de focalisation numérique sont connus de l'homme du métier. L'image reconstruite $I_Z$ forme l'image d'intérêt.

Etape 250 : application d'un indicateur statistique de l'image d'intérêt $I_Z$. Cet indicateur peut être une moyenne, une médiane ou un moment tel que précédemment décrit. Il peut notamment s'agir de la variance. L'application de l'indicateur statistique $k$ permet d'obtenir l'indicateur d'amplification.

Les étapes 200 à 230 peuvent être répétées, de façon à obtenir successivement différentes images acquises par le capteur d'image. On obtient alors autant d'images complexes $A_Z(t)$, desquelles on détermine autant d'images reconstruites $I_Z(t)$ et d'indicateurs de suivi d'amplification. Cela permet l'obtention d'une évolution temporelle $k(t)$ de l'indicateur d'amplification. A l'instar de l'étape 240 précédemment décrite, il est possible de déterminer un instant seuil $t_s$ auquel l'indicateur de suivi franchit un seuil $k_{th}$, de façon à estimer une durée $\Delta t$ entre l'instant seuil $t_s$ et l'instant initial $t_0$, ce dernier correspondant au mélange entre l'échantillon et le milieu réactionnel.

La présente invention est mise en oeuvre avec un procédé d'amplification de type LAMP et nécessite formation de précipités selon une concentration supérieure à 0.5 mM ou 1 mM. D'autres procédés d'amplification sont décrits, par exemple des procédés de type PCR ou RBA (acronyme de Recombinase Polymerase Amplification) mais ne font pas partie de l'invention.

**Revendications**

1. Procédé de suivi de l'amplification in-vitro d'une séquence de nucléotides contenue dans un échantillon, l'amplification visant à répliquer la séquence de nucléotides par une enzyme ADN polymérase, l'amplification étant de type LAMP (Loop-Mediated Isothermal Amplification), le procédé comportant les étapes suivantes :

a) mélange de l'échantillon (10) avec un milieu réactionnel, apte à l'obtention de réplications multiples d'une séquence de nucléotides prédéterminée, dite séquence cible, le mélange étant réalisé à un instant initial ($t_0$);
b) illumination de l'échantillon (10) à l'aide d'une source de lumière (11) ponctuelle, la source de lumière émettant une onde lumineuse incidente (12) se propageant vers l'échantillon, dans une bande spectrale d'illumination;
c) acquisition, à l'aide d'un capteur d'image (16), d'une image bidimensionnelle ($I_0$) de l'échantillon, l'échantillon étant disposé entre la source de lumière et le capteur d'image de telle sorte que le capteur d'image est exposé à une onde lumineuse dite d'exposition (14) ayant traversé l'échantillon, l'acquisition étant réalisée à un instant d'acquisition ($t$) postérieur à l'instant initial ;
d) à partir de l'image acquise ($I_0$), formation d'une image d'intérêt ($I_0$,$ROI$,$I_Z$), représentative de la formation d'un précipité ($10_p$) dans l'échantillon sous l'effet de l'amplification;
e) détermination d'un indicateur *(k, k(t))*, dit indicateur d'amplification, en fonction d'une distribution de l'intensité des pixels dans tout ou partie de l'image d'intérêt, l'indicateur d'amplification étant représentatif d'une quantité de séquences cibles répliquée ;

le procédé étant **caractérisé en ce que** lors de l'étape c), l'image bidimensionnelle de l'échantillon est acquise dans la bande spectrale d'illumination et que lors de l'étape e), l'indicateur d'amplification est déterminé en fonction d'une dispersion de l'intensité des pixels dans l'image d'intérêt.

**2.** Procédé selon la revendication 1, dans lequel lors de l'étape d), l'image d'intérêt est formée par l'image acquise ($I_0$) ou par une région d'intérêt (ROI) de l'image acquise.

**3.** Procédé selon la revendication 1 ou la revendication 2 dans lequel l'étape d) comporte les sous-étapes suivantes:

di) à partir de tout ou partie de l'image acquise lors de l'étape c), application d'un opérateur de propagation ($h$) de façon à calculer une amplitude complexe ($A_z$) de l'onde lumineuse d'exposition (14), selon une surface de reconstruction ($P_z$) s'étendant face au capteur d'image ;
dii) à partir de l'amplitude complexe, formation d'une image, dite image reconstruite, représentative du module et/ou de la phase de l'amplitude complexe dans la surface de reconstruction, l'image reconstruite formant l'image d'intérêt ($I_z$).

**4.** Procédé selon l'une quelconque des revendications précédentes, dans lequel lors de l'étape e), l'indicateur d'amplification ($k,k(t)$) est obtenu à partir d'un moment d'ordre strictement supérieur à 1 de la distribution de l'intensité de pixels de l'image d'intérêt.

**5.** Procédé selon l'une quelconque des revendications précédentes, dans lequel l'indicateur d'amplification ($k,k(t)$) est obtenu à partir d'une variance ou d'un écart type de la distribution de l'intensité de pixels de l'image d'intérêt.

**6.** Procédé selon l'une quelconque des revendications précédentes, dans lequel les étapes a) à e) sont effectuées à différents instants d'acquisition, le procédé comportant alors une étape f) de suivi temporel de la valeur ($k(t)$) de l'indicateur d'amplification auxdits instants d'acquisition.

**7.** Procédé selon l'une quelconque des revendications précédentes, comportant une étape g) de détection d'un instant, dit instant seuil ($t_s$), auquel l'indicateur d'amplification franchit une valeur seuil ($k_{th}$), de façon à obtenir une durée ($\Delta t$) entre l'instant seuil ($t_s$) et l'instant initial ($t_0$).

**8.** Procédé selon l'une quelconque des revendications précédentes, dans lequel l'échantillon est contenu dans une chambre fluidique (15) dont l'épaisseur est inférieure à 1 mm, la surface de l'échantillon observée par le capteur d'image étant supérieure à 10 mm$^2$.

**9.** Procédé selon l'une quelconque des revendications précédentes, dans lequel l'onde lumineuse d'exposition (14) comporte une partie de l'onde lumineuse incidente (12') ayant traversé l'échantillon ainsi qu'une onde de diffraction (13) résultant de la diffraction par l'échantillon de l'onde lumineuse incidente (12) sous l'effet de la formation du précipité (10p) dans l'échantillon.

**10.** Procédé selon l'une quelconque des revendications précédentes, dans lequel aucune optique de grossissement n'est disposée entre le capteur d'image (16) et l'échantillon (10).

**11.** Procédé selon l'une quelconque des revendications précédentes, dans lequel la source de lumière (11) est spatialement cohérente.

**12.** Dispositif pour le suivi de l'amplification in-vitro d'une séquence de nucléotides dans un échantillon, le dispositif comportant :

- une source de lumière ponctuelle (11) apte à émettre une onde lumineuse incidente (12) se propageant vers l'échantillon (10) ;
- un support (10s), configuré pour maintenir l'échantillon (10) entre la source de lumière (11) et un capteur d'image (16) ;

un processeur (20), configuré pour recevoir une image de l'échantillon acquise par le capteur d'image (16) et à mettre en oeuvre les étapes c) à e) du procédé objet de l'une quelconque des revendications 1 à 13.

**13.** Dispositif selon la revendication 12, dans lequel aucune optique de formation d'image n'est disposée entre le capteur d'image et le support.

**Patentansprüche**

**1.** Verfahren zur Nachverfolgung der In-vitro-Amplifikation einer in einer Probe enthaltenen Nukleotidsequenz, wobei die Amplifikation darauf abzielt, die Nukleotidsequenz durch ein DNA-Polymerase-Enzym zu replizieren, die Amplifikation vom LAMP-Typ (Loop-Mediated Isothermal Amplification) ist, wobei das Verfahren die folgenden Schritte umfasst:

a) das Vermischen der Probe (10) mit einem Reaktionsmedium, das zum Erhalt von mehreren Replikationen einer vorbestimmten, als Zielsequenz bezeichneten Nukleotidsequenz geeignet ist, wobei die Mischung zu einem Anfangszeitpunkt ($t_0$) durchgeführt wird;
b) die Beleuchtung der Probe (10) mit einer Punktlichtquelle (11), wobei die Lichtquelle in einem Beleuchtungsspektralband eine einfallende Lichtwelle (12) ausstrahlt, die sich in Richtung der Probe fortpflanzt;

c) die Erfassung eines zweidimensionalen Bildes ($I_0$) der Probe mittels eines Bildsensors (16), wobei die Probe zwischen der Lichtquelle und dem Bildsensor so angeordnet ist, dass der Bildsensor durch eine als Belichtung (14) bezeichnete Lichtwelle, welche die Probe durchquert hat, belichtet wird, wobei die Erfassung zu einem Erfassungszeitpunkt ($t$) nach dem Anfangszeitpunkt erfolgt;

d) ausgehend vom erfassten Bild ($I_0$) die Bildung eines Bildes ($I_0, ROI, I_z$) von Interesse, das repräsentativ für die Bildung eines Niederschlags ($10_p$) in der Probe unter Einwirkung der Amplifikation ist;

e) die Bestimmung eines als Amplifikationsindikator bezeichneten Indikators ($k, k(t)$) in Abhängigkeit von einer Verteilung der Pixelintensität im gesamten Bild oder einem Teil des Bildes von Interesse, wobei der Amplifikationsindikator repräsentativ für eine Menge von replizierten Zielsequenzen ist;

wobei das Verfahren **dadurch gekennzeichnet ist, dass** in Schritt c) das zweidimensionale Bild der Probe in einem Beleuchtungsspektralband erfasst wird und in Schritt e) der Amplifikationsindikator in Abhängigkeit von einer Verteilung der Pixelintensität im Bild von Interesse bestimmt wird.

2. Verfahren nach Anspruch 1, wobei in Schritt d) das Bild von Interesse durch das erfasste Bild ($I_0$) oder durch einen Bereich ($ROI$) von Interesse des erfassten Bildes gebildet wird.

3. Verfahren nach einem der Ansprüche 2 oder 3, wobei Schritt d) die folgenden Teilschritte umfasst:

di) ausgehend vom gesamten Bild oder einem Teil des in Schritt c) erfassten Bildes die Anwendung eines Ausbreitungsoperators ($h$) so, dass eine komplexe Amplitude ($A_z$) der Belichtungslichtwelle (14) gemäß einer Rekonstruktionsfläche ($P_z$) berechnet wird, die sich gegenüber dem Bildsensor erstreckt;

dii) ausgehend von der komplexen Amplitude die Bildung eines als rekonstruiertes Bild bezeichneten Bildes, das repräsentativ für den Modul und/oder die Phase der komplexen Amplitude in der Rekonstruktionsfläche ist, wobei das rekonstruierte Bild das Bild ($I_Z$) von Interesse bildet.

4. Verfahren nach einem der vorhergehenden Ansprüche, wobei in Schritt e) der Amplifikationsindikator ($k, k(t)$) ausgehend von einem Moment einer Ordnung strikt größer als 1 der Verteilung der Pixelintensität des Bildes von Interesse erhalten wird.

5. Verfahren nach einem der vorhergehenden Ansprüche, wobei der Amplifikationsindikator ($k, k(t)$) ausgehend von einer Varianz oder einer Standardabweichung der Verteilung der Pixelintensität des Bildes von Interesse erhalten wird.

6. Verfahren nach einem der vorhergehenden Ansprüche, wobei die Schritte a) bis e) zu verschiedenen Erfassungszeitpunkten durchgeführt werden, wobei das Verfahren dann einen Schritt f) der zeitlichen Nachverfolgung des Wertes ($k(t)$) des Amplifikationsindikators zu den Erfassungszeitpunkten umfasst.

7. Verfahren nach einem der vorhergehenden Ansprüche, das einen Schritt g) der Detektion eines als Schwellenzeitpunkt ($t_s$) bezeichneten Zeitpunkts umfasst, zu dem der Amplifikationsindikator einen Schwellenwert ($k_{th}$) überschreitet, sodass ein Zeitraum ($\Delta t$) zwischen dem Schwellenzeitpunkt ($t_s$) und dem Anfangszeitpunkt ($t_0$) erhalten wird.

8. Verfahren nach einem der vorhergehenden Ansprüche, wobei die Probe in einer Fluidkammer (15) enthalten ist, deren Dicke weniger als 1 mm beträgt, wobei die vom Bildsensor beobachtete Probenfläche größer als 10 mm$^2$ ist.

9. Verfahren nach einem der vorhergehenden Ansprüche, wobei die Belichtungslichtwelle (14) einen Teil der einfallenden Lichtwelle (12'), der die Probe durchquert hat, sowie eine gebeugte Welle (13) umfasst, die aus der Beugung der einfallenden Lichtwelle (12) durch die Probe unter Einwirkung der Bildung des Niederschlags (10p) in der Probe resultiert.

10. Verfahren nach einem der vorhergehenden Ansprüche, wobei zwischen dem Bildsensor (16) und der Probe (10) keine Vergrößerungsoptik angeordnet ist.

11. Verfahren nach einem der vorhergehenden Ansprüche, wobei die Lichtquelle (11) räumlich kohärent ist.

12. Vorrichtung zur Nachverfolgung der In-vitro-Amplifikation einer Nukleotidsequenz in einer Probe, wobei die Vorrichtung Folgendes umfasst:

- eine Punktlichtquelle (11), die zum Ausstrahlen einer einfallenden Lichtwelle (12) geeignet ist, die sich in Richtung der Probe (10) fortpflanzt;
- eine Halterung (10s), die so ausgebildet ist, dass die Probe (10) zwischen der Lichtquelle (11) und einem Bildsensor (16) gehalten wird;

einen Prozessor (20), der so ausgebildet ist, dass er ein vom Bildsensor (16) erfasstes Bild der Probe empfängt und die Schritte c) bis e) des Verfahrens nach einem der Ansprüche 1 bis 11 ausführt.

13. Vorrichtung nach Anspruch 12, wobei zwischen dem Bildsensor und der Halterung keine Bilderzeugungsoptik angeordnet ist.

## Claims

1. Method for tracking the in-vitro amplification of a nucleotide sequence contained in a sample, the amplification aiming to replicate the nucleotide sequence via a polymerase DNA enzyme, the amplification being loop-mediated isothermal amplification, also known by its acronym LAMP, the method including the following steps:

    a) mixing the sample (10) with a reaction medium that is apt to obtain multiple replications of a preset nucleotide sequence called the target sequence, the mixing being carried out at an initial time ($t_0$);
    b) illuminating the sample (10) using a point light source (11), the light source emitting, in an illumination spectral band, an incident light wave (12) that propagates towards the sample;
    c) acquiring, using an image sensor (16), a two-dimensional image ($I_0$) of the sample, the sample being placed between the light source and the image sensor such that the image sensor is exposed to what is called an exposure light wave (14) having passed through the sample, the acquisition being carried out at an acquisition time ($t$) subsequent to the initial time;
    d) on the basis of the acquired image ($I_0$), forming an image of interest ($I_0$, $ROI$, $I_Z$) that is representative of the formation of a precipitate ($10_p$) in the sample under the effect of the amplification; and
    e) determining an indicator $(k, k(t))$, called the amplification indicator, depending on a distribution of the intensity of the pixels in all or some of the image of interest, the amplification indicator being representative of a replicated amount of target sequences;

    the method being **characterized in that**, in step c), the two-dimensional image of the sample is acquired in the illumination spectral band and **in that**, in step e), the amplification indicator is determined depending on a dispersion of the intensity of the pixels in the image of interest.

2. Method according to Claim 1, wherein, in step d), the image of interest is formed by the acquired image ($I_0$) or by a region of interest ($ROI$) of the acquired image.

3. Method according to any one of Claims 1 or 2, wherein step d) includes the following substeps:

    di) to all or some of the image acquired in step c), applying a propagation operator ($h$) so as to calculate a complex amplitude ($A_Z$) of the exposure light wave (14), on a reconstruction surface ($P_Z$) lying facing the image sensor; and
    dii) on the basis of the complex amplitude, forming an image, called the reconstructed image, representative of the modulus and/or phase of the complex amplitude on the reconstruction surface, the reconstructed image forming the image of interest ($I_Z$).

4. Method according to any one of the preceding claims, wherein, in step e), the amplification indicator $(k, k(t))$ is obtained from a moment of order strictly higher than 1 of the distribution of the pixel intensity of the image of interest.

5. Method according to any one of the preceding claims, wherein the amplification indicator $(k, k(t))$ is obtained from a variance or a standard deviation of the distribution of the pixel intensity of the image of interest.

6. Method according to any one of the preceding claims, wherein steps a) to e) are carried out at various acquisition times, the method then including, in a step f), temporally tracking the value ($k(t)$) of the amplification indicator at said acquisition times.

7. Method according to any one of the preceding claims, including a step g) of detecting a time, called the threshold time ($t_S$), at which the amplification indicator passes a threshold value ($k_{th}$), so as to obtain a length of time ($\Delta t$) between the threshold time ($t_S$) and the initial time ($t_0$).

8. Method according to any one of the preceding claims, wherein the sample is contained in a fluidic chamber (15) the thickness of which is smaller than 1 mm, the area of the sample observed by the image sensor being larger than 10 mm$^2$.

9. Method according to any one of the preceding claims, wherein the exposure light wave (14) includes an incident light-wave portion (12') having passed through the sample and a diffracted wave (13) resulting from diffraction by the sample of the incident light wave (12) under the effect of the formation of the precipitate ($10_p$) in the sample.

10. Method according to any one of the preceding claims, wherein no magnifying optics are placed between the image sensor (16) and the sample (10).

**11.** Method according to any one of the preceding claims, wherein the light source (11) is spatially coherent.

**12.** Device for tracking the in-vitro amplification of a sequence of nucleotides in a sample, the device including:

- a point light source (11) able to emit an incident light wave (12) that propagates towards the sample (10);
- a holder (10s), configured to hold the sample (10) between the light source (11) and an image sensor (16); and
- a processor (20), configured to receive an image of the sample, which image is acquired by the image sensor (16), and to implement steps c) to e) of the method according to any one of Claims 1 to 11.

**13.** Device according to Claim 12, wherein no image-forming optics are placed between the image sensor and the holder.

**Fig. 1**

**Fig. 2A**

**Fig. 2B**

**Fig. 3A**

**Fig. 3B**

**Fig. 3C**

**Fig. 3D**

**Fig. 3E**

**Fig. 4A**

**Fig. 4B**

**Fig. 4C**

**Fig. 5A**

**Fig. 5B**

**Fig. 5C**

**Fig. 5D**

**Fig. 5E**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1652500

**Littérature non-brevet citée dans la description**

- **NOTOMI T.** Loop-mediated isothermal amplification of DNA. *Nucleic Acids Research,* 2000, vol. 28 (12
- **HILL JOSHUA.** Loop-Mediated Isothermal Amplification Assay for Rapid Detection of Common Strains of Escherichia coli. *Journal of Clinical Microbiology,* Août 2008, 2800-2804
- **WANG T. et al.** *Real-time DNA Amplification and Détection System based on a CMOS Image sensor*
- **MORI Y.** Real-time turbidimetry of LAMP reaction for quantifying template DNA. *J. Biochem. Biophys. Methods,* 2004, vol. 59, 145-157
- **MORI Y.** Détection of Loop-Mediated Isothermal Amplification Reaction by Turbidity Derived from Magnesium Pyrophosphate Formation. *Biochemical and Biophysical Research Communications,* 2001, vol. 289, 150-154